# DEMANDE DE BREVET EUROPEEN

(11) **EP 1 155 675 A2**
(43) Date de publication de la demande: **21.11.2001**
(21) Numéro de dépôt: 01401248.8
(22) Date de dépôt: 15.05.2001
(51) Int. Cl.: A61K 7/02

(54) **Composition de maquillage comprenant une phase liante particulière**

(30) Priorité: 19.05.2000 FR 0006451
(71) Demandeur: L'OREAL, 75008 Paris (FR)
(72) Inventeur: Grimm, Sabine, 92290 Châtenay-Malabry (FR); Hadasch, Anke, 75005 Paris (FR); Terren-Gardel, Nadia, 92340 Bourg-La-Reine (FR)
(74) Mandataire: Doressamy, Clarisse

(57) **Abrégé**

La présente invention est relative à une composition de maquillage comprenant une phase pulvérulente et une phase liante, caractérisée par le fait que la phase liante comprend une dispersion de particules de gel cubique.

Elle porte également sur les applications cosmétiques d'une telle composition.

## Description

La présente invention a pour objet une composition de maquillage comprenant une phase liante particulière. Ces compositions peuvent être de produits pour le teint, notamment des poudres, par exemple libre, compacte, pressée ou encore coulée, ou des fonds de teint, ou encore des produits pouvant jouer à la fois le rôle d'une poudre et d'un fond de teint, ou des produits de maquillage du corps. Ces compositions peuvent également être des produits pour les lèvres comme des rouges à lèvres, de produits pour les yeux comme des mascaras ou des eye-liner. Elles peuvent se présenter sous la forme de produits coulés, de crèmes, de poudres, etc...

Les produits de maquillage comprennent généralement, d'une part, une phase pulvérulente comportant notamment des pigments et des charges et d'autre part, une phase liante ou dispersante qui comprend généralement des corps gras, destinée à disperser lesdits composés pulvérulents de façon homogène et/ou à faciliter leur cohésion entre eux au sein de la composition finale. Cette phase liante est également destinée à conférer au produit fini une certaine densité, à donner une douceur et une propriété émolliente au produit de maquillage et à favoriser son adhérence sur la peau.

L'élaboration des phases liantes ou dispersantes dans les produits de maquillage peut soulever de nombreuses difficultés.

En ce qui concerne plus particulièrement les poudres, la composition finale doit être suffisamment homogène et compacte pour présenter une bonne aptitude au prélèvement et pour éviter par ailleurs une fragmentation provoquée notamment par des chocs.

Il est possible d'utiliser comme phase liante dans les poudres un mélange d'huiles minérales et végétales associées à des esters. Cependant, les produits contenant ces esters peuvent mener à des produits manquant de douceur, voire à une mauvaise dispersion de la phase pulvérulente.

Il est également possible d'utiliser des huiles de silicone qui peuvent apporter du glissant, de la douceur, de la facilité d'étalement mais les propriétés de tenue du maquillage et de résistance au choc peuvent s'avérer médiocres.

Enfin, certaines huiles perfluorées, en particulier les perfluoropolyéthers, sont bien connues comme apportant de la douceur aux compositions cosmétiques comme les poudres, mais le formulateur se trouve alors confronté à des problèmes d'insolubilité de ces huiles dans les huiles hydrocarbonées usuelles ou les huiles de silicones, cette insolubilité entraînant des problèmes d'instabilité de la composition de maquillage et/ou de soin. Par ailleurs, les compositions comprenant des huiles perfluorées présentent de médiocres propriétés de résistance au choc. Certaines de ces huiles peuvent également présenter l'inconvénient d'une mauvaise dispersion de la phase pulvérulente : la teinte apportée par le pigment est relativement peu intense, pouvant provoquer un « effet blanc », ce qui n'est pas souhaité, esthétiquement parlant.

Ainsi, les poudres de maquillage sont souvent des produits constitués généralement d'un très fort taux de composés pulvérulents que l'on pourrait qualifier de « secs » et d'huile. Ces produits peuvent ainsi donner des sensations de tiraillement ou un effet desséchant lorsqu'ils sont appliqués sur la peau. Toutefois, du fait du fort taux de composés pulvérulents, il est très difficile d'introduire de l'eau dans de tels produits. En effet, l'eau ne lie pas les particules, elle s'agglomère avec les composés pulvérulents pour former une sorte de pâte hétérogène et instable ou gâchage dont l'utilisation en tant que produit de maquillage est impossible : un prélèvement dosé de produit ne peut pas se faire. Par ailleurs, on n'obtient plus un produit poudré destiné à donner au teint un aspect velouté mais une pâte collante donnant un effet plâtre.

Un autre inconvénient de la difficulté d'introduire de l'eau dans les poudres de maquillage est l'impossibilité d'incorporer dans les poudres des agents actifs, en particulier hydrophiles, tout en conservant une composition stable.

Or, de plus en plus, les utilisatrices souhaitent des produits de maquillage qui non seulement les embellissent mais qui apportent également de la fraîcheur ou du soin à leur peau.

En ce qui concerne plus particulièrement les fonds de teint, les rouge-à-lèvres, les mascaras, les eye-liners ou les produits de maquillage du corps, la composition finale doit également être homogène, les composés pulvérulents doivent être bien dispersés afin de rendre à l'application un maquillage uniforme sur la peau.

Afin d'optimiser la dispersion des composés pulvérulents dans ces produits, on peut par exemple préparer une pâte préalable constituée des pigments et de corps gras particulièrement aptes à disperser lesdits pigments, puis ajouter ensuite le reste des composés de la composition. On peut utiliser également des appareils mixeur-mélangeur qui à l'aide de pales ou de turbines dispersent efficacement les composés pulvérulents. Il est pratiquement impossible de bien disperser les composés pulvérulents dans un fond de teint par exemple par des moyens primaires, du type spatule ou banal agitateur magnétique.

Il est bien sûr déjà connu d'incorporer de l'eau dans des fonds de teint, des mascaras, des eye-liners voire dans des rouge-à-lèvres : de tels produits peuvent se présenter classiquement sous la forme d'émulsions E/H ou H/E. Toutefois, dans de tels produits, la dispersion des composés pulvérulents, en particulier des pigments n'est pas toujours optimale. Par ailleurs, on cherche également à introduire das ces produits, en particulier dans les fonds de teint, des actifs de soin tout en conservant une composition stable et homogène, dont les actifs ne sont pas oxydés par le pigments.

Ainsi, il serait particulièrement utile de pouvoir réaliser des produits de maquillage, en particulier des produits pour le teint tels que les poudres et les fonds de teint, comprenant une phase liante qui non seulement disperserait les composés pulvérulents de manière optimale mais qui de plus permettrait de véhiculer des actifs et de procurer un effet fraîcheur sur la peau.

La Demanderesse a trouvé de manière inattendue que l'utilisation d'une composition particulière comme comme phase liante ou dispersante, cette composition particulière étant une dispersion de particules de gel cubique, permettait l'obtention de produits de maquillage présentant une excellente dispersion des pigments tout en apportant de la fraîcheur et de l'hydratation.

L'invention a donc pour objet une composition de maquillage comprenant une phase pulvérulente et une phase liante, caractérisée par le fait que la phase liante comprend une dispersion de particules de gel cubique.

Les compositions ainsi obtenues provoquent une agréable sensation de fraîcheur à l'application. Elles hydratent la peau et ne procurent pas d'effet de tiraillement ou de dessèchement. Elles présentent également une excellente dispersion des pigments. La composition obtenue est très homogène et elle le reste même après application sur la peau, et ce pendant plusieurs heures.

Les compositions sous forme de poudre ainsi obtenues présentent une excellente tenue. Elles ne transfèrent pas et ne migrent pas non plus dans les plis de la peau.

En particulier, grâce à cette phase liante particulière, il est maintenant possible de réaliser des compositions de fonds de teint d'une manière extrêmement simple. Par exemple, il est possible de réaliser un fond de teint homogène en mettant seulement un peu de phase liante ou dispersante dans le creux de la main puis d'y ajouter des composés pulvérulents comme par exemple des pigments. En mélangeant simplement les pigments à l'aide d'un doigt, on obtient très facilement une compsoition colorée dans laquelle les pigments sont très bien dispersés. La composition obtenue dans le creux de la main peut être directement utilisée, comme un fond de teint classique, en l'appliquant et en l'étalant sur la peau. Le maquillage ainsi effectué est homogène et uniforme et ne forme pas de plaques sur le visage comme c'est le cas si l'on utilise de la même manière une phase liante classique. Ainsi, n'importe quelle utilisatrice ayant à sa disposition un flacon de phase liante selon l'invention et un panel de godets comprenant des pigments et/ou des charges et/ou des actifs sous forme pulvérulente est capable de fabriquer son propre fond de teint à la carte, lorsqu'elle le désire et dans la quantité qu'elle souhaite.

Les compositions de fonds de teint obtenues présentent une excellente tenue (pas de virage de la couleur) ainsi que de la matité en transparence.

On peut également réaliser de la même manière des compositions de rouge-à-lèvres, de mascaras, d'eye-liners, de produits de maquillage du corps, etc...

Les compositions selon l'invention présentent également d'excellentes propriétés cosmétiques : elles adhèrent suffisamment à la peau mais pas trop, elles sont très douces, elles s'appliquent facilement.

La présente invention a encore pour objet un procédé cosmétique de maquillage ou de soin, de préférence de maquillage, des matières kératiniques des êtres humains, en particulier de la peau et du corps, comprenant l'application sur ces matières de la composition telle que définie ci-dessus.

Par matières kératiniques, on entend selon la présente invention, la peau, les ongles, les cheveux, les cils et sourcils, les muqueuses (intérieurs des paupières inférieures) et les semi-muqueuses (lèvres), et toute autre zone cutanée du corps et du visage.

La présente invention a encore pour objet l'utilisation d'au moins une dispersion de particules de gel cubique, dans une composition de maquillage comprenant des composés pulvérulents, dans le but d'améliorer la dispersion desdits composés pulvérulents dans ladite composition. Une telle dispersion a généralement une fonction de dispersion desdits composés pulvérulents.

La présente invention a encore pour objet l'utilisation d'au moins une dispersion de particules de gel cubique, dans une composition de maquillage, dans le but d'améliorer l'hydratation sur les matières kératiniques conférée par ladite composition. Une telle dispersion a généralement une fonction d'hydratation desdites matières kératiniques.

La présente invention a également pour objet un kit de maquillage comprenant d'une part un récipient comprenant une composition comprenant au moins une dispersion de particules de gel cubique et d'autre part au moins un récipient séparé comprenant un composé pulvérulent.

Les compositions selon l'invention trouvent notamment une application particulièrement intéressante dans le domaine du maquillage et/ou du soin des matières kératiniques des êtres humains, en particulier de la peau et des muqueuses. Ainsi, l'invention trouve une application toute particulière dans le domaine des produits de maquillage du visage et de la peau, tels que les fards à paupières, les poudres du visage et du corps, les anticernes, les fonds de teint, les produits de maquillage du corps, les rouge-à-lèvres, les mascaras, les eye-liners ainsi que les produits solaires ou autobronzants.

D'autres caractéristiques, aspects et avantages de la présente invention apparaîtront à la lecture de la description détaillée qui va suivre.

Les compositions selon l'invention comprennent une phase pulvérulente qui peut comprendre des pigments et/ou des nacres et/ou des charges et/ou des paillettes habituellement utilisés dans les compositions cosmétiques et/ou leurs mélanges.

Par pigments, il faut comprendre des particules blanches ou colorées, minérales ou organiques, insolubles dans le milieu, destinées à colorer et/ou opacifier la composition.

Les pigments peuvent être présents à une teneur allant de 0,05 à 80 % en poids, par rapport au poids total de la composition, et de préférence allant de 0,5 à 50 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, les nanozincs, le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des polydiméthylsiloxanes et/ou par des polymères, notamment des polyéthylènes. On peut ainsi citer les pigments SI vendus par la société Miyoshi.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes dans la composition à une teneur allant de 0,05 à 99 % en poids, par rapport au poids total de la composition, peuvent être minérales ou de synthèse, lamellaires ou non lamellaires. On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques telles que l'Expancel (Nobel Industrie), les microéponges comme le polytrap (Dow Corning) et les microbilles de résine de silicone (Tospearls de la Société TOSHIBA, par exemple), les oxydes de zinc et de titane, les oxydes de zirconium ou de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre ou de céramique ; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Par nacres, il faut comprendre des particules irisées qui réfléchissent la lumière.

Les nacres peuvent être présentes dans la composition à une teneur allant de 0,05 à 80% en poids, de préférence à une teneur allant de 2 à 50% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les compositions selon l'invention peuvent également comprendre des paillettes.

De préférence, la phase pulvérulente est présente dans les compositions selon l'invention à une teneur d'au moins 0,1% en poids, de préférence encore d'au moins 2% en poids, par rapport au poids total de la composition.

Les compositions selon l'invention comprennent une phase liante, ou encore dispersante, particulière. Cette phase liante est une composition, de préférence à phase continue aqueuse, dans laquelle sont dispersées des particules de gel cubique.

Le terme de gel cubique utilisé selon la présente invention désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans « La Recherche », Vol.23, pp. 306-315, Mars 1992 et dans « Lipid Technology », Vol.2, n° 2, pp.42-45, Avril 1990. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

De tels gels cubiques sont par exemple décrits dans EP 0 686 386 et EP 0 711 540.

Dans une première forme préférée de réalisation de l'invention, la phase liante est sous la forme d'une dispersion, à phase continue aqueuse , de particules de gel cubique à base de phytantriol comprenant :
(a) de 0,1 à 15 % en poids de 3,7,11,15-tétraméthyl 1,2,3-hexadecanetriol ou phytantriol par rapport au poids total de phase liante, et
(b) de 0,1 à 3 % en poids d'un agent dispersant et stabilisant par rapport au poids total de la phase liante, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

Selon un mode de réalisation préféré, la proportion en phytantriol est comprise entre 0,5 et 10 % en poids par rapport au poids total de la phase liante.

De préférence, le rapport pondéral entre le phytantriol et ledit agent dispersant et stabilisant tel que défini précédemment est compris entre 1 et 200, et de façon particulièrement préférée, entre 2 et 50.

Le phytantriol est un composé connu qui est notamment commercialisé sous la dénomination de « Phytantriol-63926 »® par la Société ROCHE.

Dans une deuxième forme préférée de l'invention, la phase liante est une composition sous forme d'une dispersion comprenant :
(α) de 60 à 98 % en poids d'une phase aqueuse, et
(β) de 2 à 40 % en poids d'une phase huileuse,
ladite phase huileuse étant dispersée dans ladite phase aqueuse et stabilisée à l'aide de particules de gel cubique, lesdites particules étant essentiellement formées par :
(i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et
(ii) 0,05 à 3 % en poids par rapport au poids total de la composition d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

De préférence, la proportion pondérale relative en composé (i) par rapport au poids de la phase huileuse est comprise entre 0,02/1 et 1/1, et de préférence comprise entre 0,05/1 et 0,5/1.

Selon un mode de réalisation particulier des compositions selon l'invention, la proportion pondérale relative en composé (i) par rapport au poids dudit agent dispersant et stabilisant est comprise entre 2 et 200, et de préférence inférieure ou égale à 50.

Parmi les dérivés N-2-alcoxycarbonyles de N-méthylglucamine, on peut notamment citer ceux répondant à la formule (I) suivante : 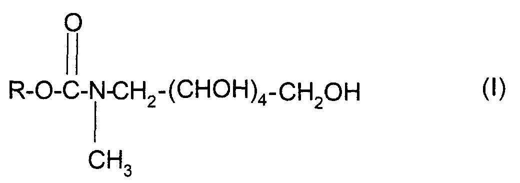 dans laquelle :
R représente un radical alkyle ramifié en C₆-C₁₈.

Parmi ceux-ci, on peut notamment mentionner la N-2-hexyldécyloxycarbonyl-N-méthyl-glucamine, la N-2-éthylhexyloxycarbonyl-N-méthyl-glucamine et la N-2-butyloctyloxycarbonyl-N-méthyl-glucamine.

La méthode de préparation des composés de formule (I) est décrite dans EP 0 711 540.

Selon un mode de réalisation particulier des phases liantes selon l'invention, les particules de gel cubique contiennent comme composé (i), un mélange de phytantriol en une proportion de 1 à 40 % en poids par rapport au poids du mélange, et d'au moins un dérivé N-2-alcoxycarbonyle de N-méthyl-glucamine de formule (I) en une proportion de 60 à 99 % en poids par rapport au poids du mélange.

Selon une forme préférée de ce mode de réalisation, la proportion en phytantriol est de 10 à 30 % en poids par rapport au poids du mélange et celle du dérivé N-2-alcoxycarbonyle de N-méthyl-glucamine de 70 à 90 % en poids par rapport au poids du mélange.

Les monoglycérides d'acide gras insaturé sont de préférence ceux ayant une chaîne grasse insaturée en C₁₆-C₂₂.

Parmi ceux-ci, on peut notamment citer le monooléate de glycéryle ou monooléine et le monolinoléate de glycéryle ou monolinoléine.

On peut bien entendu utiliser dans les compositions selon l'invention, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Selon un autre mode de réalisation des phases liantes selon l'invention, les particules de gel cubique contiennent comme composé (i), un mélange de phytantriol en une proportion de 1 à 50 % en poids par rapport au poids total du mélange et d'au moins un monoglycéride d'acide gras insaturé en une proportion de 50 à 99 % en poids par rapport au poids du mélange.

Selon une forme préférée de ce mode de réalisation, la proportion en phytantriol est de 10 à 30 % en poids par rapport au poids du mélange et celle en monoglycéride d'acide gras insaturé de 70 à 90 % en poids par rapport au poids du mélange.

L'agent dispersant et stabilisant tel que défini précédemment est de préférence choisi parmi :
- les alkyl ou alcényl éthers ou esters de polyol,
- les amino-acides N-acylés et leurs dérivés et les peptides N-acylés par un radical alkyle ou alcényle, et leurs sels,
- les alkyl ou alcényl éthers ou esters sulfates, leurs dérivés et leurs sels,
- les alkyl ou alcényl éthers ou esters gras polyoxyéthylénés,
- les acides alkyl ou alcényl carboxyliques polyoxyéthylénés et leurs sels,
- les N-alkyl ou alcényl bétaïnes,
- les alkyl ou alcényl triméthylammonium et leurs sels, et
- leurs mélanges.

Dans les composés ci-dessus énumérés, les radicaux alkyle et alcényle ont de 8 à 22 atomes de carbone et peuvent se présenter sous forme de mélanges.

Parmi les alkyl ou alcényl éthers ou esters de polyol, on peut citer notamment les alkyl ou alcényl esters de sorbitan polyoxyéthylénés par au moins 20 motifs d'oxyde d'éthylène tels que le palmitate de sorbitan 20 OE ou Polysorbate 40 commercialisé sous la dénomination de « Montanox 40 DF »® par la Société SEPPIC, et le laurate de sorbitan 20 OE ou Polysorbate 20 commercialisé sous la dénomination de « Tween 20 »® par la Société ICI.

Dans ce groupe, on peut également citer les alkyl ou alcényl esters de polyglycérol comportant au moins 10 motifs dérivés du glycérol, oxyéthylénés ou non, tels que le polyglycéryl-10 laurate commercialisé sous la dénomination de « Decaglyn 1-L »® par la Société NIKKO CHEMICALS.

On peut encore citer les alkyl ou alcényl éthers ou esters de mono ou polysaccharides tels que ceux dérivant du glucose, du fructose, du galactose, du maltose ou du lactose et notamment les monoesters en 1- et 6- du D-fructose, du décylglucose et du décylpolyglucose.

Parmi les amino-acides N-acylés et leurs dérivés, les peptides N-acylés par un radical alkyle ou alcényle et leurs sels, on utilise de préférence ceux pour lesquels le radical alkyle ou alcényle a au moins 12 atomes de carbone.

Par amino-acides, on entend selon l'invention les α, β ou γ-amino-acides. Comme sels d'amino-acides N-acylés, on peut citer par exemple ceux de glutamate N-acylé tels que le cocoyl glutamate de monosodium, le lauroyl glutamate de mono-sodium, l'alcoyl C₁₄-C₂₀ glutamate de disodium (le radical alcoyle C₁₄-C₂₀ dérivant du suif hydrogéné), commercialisés respectivement sous les dénominations de « Acylglutamate CS-11 »®, de « Acylglutamate LS-11 »® et de « Acylglutamate HS-21 »® par la Société AJINOMOTO.

On peut encore citer les lysines N-acylées telles que la lauroyl lysine commercialisée sous la dénomination de « Amihope LL »® par la Société AJINOMOTO.

Les dérivés d'amino-acides N-acylés et leurs sels sont de préférence les sarcosinates N-acylés tels que le lauroyl sarcosinate de sodium commercialisé sous la dénomination de « Oramix L30 »® par la Société SEPPIC, le myristoyl sarcosinate de sodium et le palmitoyl sarcosinate de sodium commercialisés respectivement sous les dénominations de « Nikkol Sarcosinate MN »® et de « Nikkol Sarcosinate PN »® par la Société NIKKO CHEMICALS.

Parmi les peptides N-acylés on peut citer ceux dérivés de tout ou partie du collagène ou de la kératine tels que le lauroyl collagène de sodium et la palmitoyl kératine commercialisés sous les dénominations de « Proteol B 30»® et de « Lipacide PK »® par la Société SEPPIC.

Parmi les alkyl ou alcényl éthers ou esters sulfates, leurs dérivés et leurs sels, on utilise de préférence ceux pour lesquels le radical alkyle ou alcényle a au moins 12 atomes de carbone.

Parmi les alkyl ou alcényl éthers sulfates, on utilise de préférence les sels d'alkyl éther sulfate et notamment le lauryléther sulfate de sodium.

Parmi les alkyl ou alcényl esters sulfates, on peut citer par exemple les esters de l'acide iséthionique ainsi que ses sels et notamment le cocoyl iséthionate de sodium commercialisé sous la dénomination de « Geropon AC 78 »® par la Société RHÔNE-POULENC.

Parmi les alkyl ou alcényl éthers ou esters gras polyoxyéthylénés, on utilise de préférence ceux pour lesquels le radical alkyle ou alcényle a au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.

Parmi les acides alkyl ou alcényl carboxyliques polyoxyéthylénés et leurs sels, on utilise de préférence ceux comportant au moins 10 motifs d'oxyde d'éthylène tels que par exemple l'acide laureth-10 carboxylique et l'acide oleth-10 carboxylique.

Parmi les N-alkyl ou alcényl bétaïnes, on utilise de préférence celles pour lesquelles le radical alkyle ou alcényle a au moins 12 atomes de carbone telles que par exemple la lauryl amidopropyl bétaïne et l'oléyl amidopropyl bétaïne.

Parmi les alkyl ou alcényl triméthylammonium et leurs sels, on utilise de préférence ceux pour lesquels le radical alkyle ou alcényle a au moins 12 atomes de carbone. Comme sels on utilise de préférence les bromures et chlorures tels que le chlorure de cocoyltriméthylammonium et le bromure de cétyltriméthylammonium.

La phase liante a un pH généralement compris entre 5 et 8 et de préférence compris entre 6 et 7.

Les phases liantes utilisables selon l'invention sont stables et peuvent être conservées pendant 2 mois à une température comprise entre 4 et 45 °C, sans présenter aucune variation d'aspect macroscopique ni microscopique, ni de couleur, ni d'odeur.

De préférence, les particules de gel cubique comprennent en outre de 0,0005 % à 5 % en poids et de préférence de 0,001 % à 2 % en poids d'un lipide amphiphile ionique non hydrosoluble.

Parmi ceux-ci, on peut notamment citer :
- les phopholipides tels que les phospholipides naturels comme la lécithine desoja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
- les esters phosphoriques d'alcool gras tels que le monocétyl phosphate et ses sels de sodium et de potassium commercialisé sous la dénomination de « Monafax 160 »® par la Société MONA, ainsi que le dimyristyl phosphate et ses sels de sodium et de potassium commercialisé sous la dénomination de « Mexoryl SY »® par la Société CHIMEX,
- les dérivés N-acylés de l'acide glutamique tels que le stéaroyl glutamate de monosodium commercialisé sous la dénomination de « Acylglutamate HS 11 »® par la Société AJINOMOTO et le mélange cocoyl-alcoyl C₁₄-C₂₀ glutamate de monosodium, le radical alcoyle C₁₄-C₂₀ dérivant du suif hydrogéné, commercialisé sous la dénomination de « Acylglutamate GS 11 »® par la Société AJINOMOTO,
- le cétylsulfate de sodium commercialisé sous la dénomination de « Nikkol SCS»® par la Société NIKKO CHEMICALS,
- le cocoyl monoglycéride sulfate de sodium commercialisé sous la dénomination de « Nikkol SGC 80 N »® par la Société NIKKO CHEMICALS, et
- les dérivés d'ammonium quaternaire tels que le chlorure de béhényltriméthylammonium, le chlorure de dilauryldiméthylammonium, le chlorure de distéaryldiméthylammonium, le méthylsulfate de 4,5-dihydro1-méthyl 2-alcoyl C₁₄-C₂₀ 1-(2-alcoyl C₁₄-C₂₀ aminoéthyl)imidazolium, les radicaux alcoyles C₁₄-C₂₀ dérivant du suif hydrogéné, commercialisé sous la dénomination de « Rewoquat W75H »® par la Société REWO CHEMISCHE, le méthylsulfate de dialkylhydroxyéthylméthylammonium dont les radicaux alkyles dérivent du suif, hydrogéné ou non, commercialisé sous la dénomination de « Stepanquat VP 85 »® par la Société STEPAN et le quaternium-82 commercialisé par la Société SEPPIC sous la dénomination de « Amonyl DM »® .

L'incorporation de ces lipides amphiphiles ioniques non hydrosolubles confère aux particules de gel cubique une charge superficielle entraînant une répulsion électrostatique des particules entre elles.

Les phases liantes sous forme de dispersion de particules de gel cubique telles que définies précédemment sont obtenues par fragmentation, à l'aide d'un homogénéiseur, d'un gel cubique à base de phytantriol, d'eau, d'au moins un agent tensioactif hydrosoluble à chaîne grasse tel que défini précédemment et éventuellement de lipides amphiphiles ioniques non hydrosolubles tels que définis précédemment et/ou de principes actifs hydrophiles et lipophiles.

Les particules de gel cubique peuvent être obtenues par divers moyens mécaniques appropriés tels que par exemple par un homogénéiseur de type rotor-stator à fort gradient de cisaillement comme le « Virtis », ou par un homogénéiseur haute pression fonctionnant entre 200 et 1.800 bars environ (20 à 180 Mpa).

La taille moyenne des particules de la dispersion, telle que définie précédemment, est généralement d'environ 0,05 à 1 µm, et de préférence inférieure ou égale à 0,5 µm. La granulométrie de la dispersion peut en outre être modulée par la nature et la concentration de l'agent tensioactif hydrosoluble à chaîne grasse utilisé.

A ce stade de la préparation, il est possible d'incorporer dans les particules de gel cubique des dispersions telles que définies précédemment, divers types de composés actifs. En particulier lesdites particules peuvent contenir un principe actif hydrophile ou un principe actif lipophile.

Bien entendu, grâce à la structure particulière des particules de gel cubique, il est possible d'incorporer dans celles-ci à la fois des principes actifs hydrophiles et des principes actifs lipophiles même si ceux-ci présentent une certaine incompatibilité.

Parmi les différents principes actifs pouvant être incorporés, on peut notamment citer :
- les agents antioxydants ou anti-radicaux libres tels que les protéines et les enzymes, la lactoperoxydase et la lactoferrine, les peptides et leurs dérivés, les séquestrants, les flavonoïdes, la chlorophylline, l'éthoxyquine, la guanosine, les tocophérols et leurs dérivés, le palmitate d'ascorbyle, le β-carotène, la vitamine E et ses dérivés, la vitamine C et ses dérivés, la vitamine A et ses dérivés,
- les agents hydratants ou humectants tels que l'acide hyaluronique et son sel de sodium, le β-glycérophosphate, le glycérol, le sorbitol, le panthénol,
- les filtres UV tels que les produits commercialisés sous les dénominations de « Eusolex 232 »® par la Société MERCK, de « Parsol 1789 »® et de « Parsol MCX »® par la Société GIVAUDAN-ROURE, de « Mexoryl SX »® par la Société CHIMEX et de « UVINUL T150 »® par la Société BASF,
- les kératolytiques tels que les enzymes protéolytiques, l'acide salicylique et ses dérivés tels que l'acide n-dodécanoyl-5 salicylique, et l'acide rétinoïque et ses dérivés,
- les accélérateurs de bronzage tels que la caféine, et les dérivés de tyrosine tels que le tyrosinate de glucose et le sel disodique de la N-L-malyl tyrosine,
- les dépigmentants tels que l'acide kojique, l'acide glycolique, la vitamine C et notamment l'ascorbyle phosphate de magnésium, et l'arbutine et ses dérivés,
- les colorants naturels tels que les matières colorantes extraites de végétaux comme la chlorophylline et le β-carotène ou extraites d'animaux comme le carmin de cochenille, et le caramel,
- les auto-bronzants tels que la dihydroxyacétone, et les indoles,
- les liporégulateurs tels que le γ-orizanol, l'extrait de *Centella asiatica* contenant de la génine et de l'acide asiatique, la caféine, et la théophylline,
- les agents anti-vieillissement et anti-rides tels que les hydroxyacides comme l'acide glycolique, l'acide n-octanoyl salicylique, le rétinol et ses dérivés comme l'acétate, le palmitate et le propionate de rétinol, et les rétinoïdes,
- les agents anti-inflammatoires et cicatrisants tels que l'acide 18 β-glycyrrhétinique et ses sels comme notamment son sel d'ammonium, l'α-bisabolol, les corticoïdes, l'extrait de *Centella asiatica,* l'aloe vera,
- les antibactériens et antifongiques tels que le chlorure de benzalkonium, la chlorhexidine, l'hexetidine, et l'hexamidine,
- les insectifuges tels que les diéthyl et diméthyltoluamides,
- les déodorants tels que l'hexachlorophène, et le triclosan produit commercialisé sous la dénomination de « Irgasan DP 300 »® par la Société CIBAGEIGY.
- les antipelliculaires tels que l'octopirox, et les dérivés de pyridinethione tels que ceux commercialisés sous les dénominations de « Omadine »® par la Société OLIN
- les agents anti-chute des cheveux tels que le nicotinate de méthyle ou d'hexyle, et le minoxidil,
- les colorants capillaires tels que les bases et les coupleurs d'oxydation, les colorants directs, et les colorants auto-oxydables,
- les agents réducteurs pour permanentes tels que l'acide thioglycolique, la cystéine, la cystéamine, la N-acétyl cystéine, la N-acétyl cystéamine, et le thioglycolate de glycérol,
- les agents conditionneurs pour peau et cheveux tels que les polymères cationiques et les cations.

Les phases liantes sous forme de dispersion selon l'invention peuvent donc comprendre soit des particules contenant des principes actifs hydrophiles, soit des particules contenant des principes actifs liphophiles, soit des particules contenant à la fois des principes actifs hydrophiles et lipophiles tels que par exemple des filtres UV hydrophiles et lipophiles, ou un mélange de ces différentes particules.

Après la formation des particules de gel cubique, l'agent dispersant et stabilisant se trouve, généralement, à l'extérieur desdites particules.

Lorsque la phase liante selon l'invention comprend une phase huileuse, on ajoute ensuite à ladite dispersion obtenue la phase huileuse contenant éventuellement certains additifs et/ou principes actifs lipophiles et on soumet le mélange à une agitation mécanique qui peut par exemple être réalisée à l'aide d'un homogénéiseur de type « Virtis® ».

La phase huileuse se trouve ainsi être dispersée dans la phase aqueuse et se présente généralement sous forme de gouttelettes de taille moyenne comprise entre 0,1 micron et 10 microns. Cette phase huileuse comprend au moins une huile d'origine végétale, animale, minérale ou de synthèse.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique.

Comme huiles de synthèse, on peut notamment citer des hydrocarbures tels que l'isohexadécane, le polydécène et le polyisobutène, des alcools gras tels que l'octyldodécanol, le cétanol, l'alcool stéarylique ou l'alcool oléique, des esters tels que les glycérides d'acides gras essentiels, les triglycérides des acides caprique et acprylique et leurs mélanges, et des esters d'alcools gras et d'acide gras linéaire ou ramifié comme l'huile de purcellin (octanoate de stéaryle).

Comme huiles de synthèse, on peut également utiliser dans les compositions selon l'invention des huiles de silicone de type linéaire telles que le polydiméthylsiloxane, de type cyclique telles que le cyclopentadiméthylsiloxane et de type organomodifié telles que le polyphényltriméthylsiloxane et le polydiméthylsiloxane oxyéthyléné oxypropyléné.

Ces huiles peuvent être utilisées seules ou en mélanges.

Il est également possible d'incorporer dans la phase aqueuse continue de la dispersion différents composés cosmétiquement ou dermatologiquement acceptables tels que des principes actifs hydrophiles comme des agents hydratants ou additifs conventionnels.

Parmi ces derniers, on peut citer notamment des agents conservateurs, des parfums, des matières colorantes, des gélifiants, etc...

Parmi les agents gélifiants pouvant être utilisés dans les compositions selon l'invention, on peut citer en particulier les dérivés de cellulose tels que l'hydroxyéthylcellulose et les alkylhydroxyéthylcelluloses, les dérivés d'algues tels que le satiagum, des gommes naturelles telles que l'adragante, des polymères synthétiques tels que les mélanges d'acides polycarboxyvinyliques et en particulier ceux commercialisés sous les dénominations de « Carbopol » par la société Goodrich ou de « Synthalen » par la société 3V SA.

La proportion en agent gélifiant va généralement de 0,1 à 2% en poids par rapport au poids total de la phase liante.

Dans une forme préférée de réalisation de l'invention, les compositions comprennent au moins un agent gélifiant.

Les compositions selon l'invention peuvent également comprendre, outre les particules dispersées de gel cubique, des liposomes contenant éventuellement des principes actifs.

La présence de phytantriol dans les compositions selon l'invention leur confère un bon pouvoir hydratant.

La phase liante peut comprendre en outre tout corps gras autre que ceux déjà cités, choisis parmi les cires et/ou les corps gras pâteux.

On peut définir les composés gras pâteux à l'aide d'au moins une des propriétés physico-chimiques suivantes :
- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 240 tours/minute,
- un point de fusion de 25-70°C, de préférence 25-55°C.

Parmi les cires utilisables, on peut citer les cires d'abeille, les cires de lanoline et les cires d'insectes de Chine ; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères cireux ainsi que leurs esters, et les cires de silicone telles que les polyalcoxy et polyalkylsiloxanes et leurs mélanges.

La phase liante peut éventuellement comprendre une partie volatile comme par exemple des huiles volatiles.

On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Ces composés volatils peuvent être choisis en particulier parmi les huiles hydrocarbonées telles que les isoparaffines et notamment l'isododécane, les silicones volatiles cycliques ayant de 3 à 8 atomes de silicium et de préférence 4 à 6, comme par exemple le cyclotétra-diméthylsiloxane, le cyclopentadiméthylsiloxane ou le cyclohexa-diméthylsiloxane, notamment les produits vendus sous les dénominations de "DC Fluid 244", "DC Fluid 245", "DC FLuid 344 et "DC Fluid 345" par la Société Dow Corning, les silicones volatiles linéaires ayant de 2 à 9 atomes de silicium, par exemple l'hexaméthyldisiloxane, l'hexylheptaméthyltrisiloxane et l'octylheptaméthyl-trisiloxane.

La phase liante peut, en outre, comprendre des additifs tels que des actifs cosmétiques lipophiles et/ou des ingrédients liposolubles généralement utilisés en cosmétique comme les parfums, les filtres solaires. De préférence, ces additifs peuvent être présents en une proportion allant de 1 à 70% en poids, par rapport au poids total de la phase liante.

Les compositions selon l'invention peuvent également comprendre des résines de silicone comprenant une combinaison des unités R₃SiO_{1/2} , R₂SiO_{2/2} , RSiO_{3/2} et SiO_{4/2}, dans lesquelles R représente l'hydrogène, un radical alkyle en C₁-C₆ ou un radical phényle.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Lorsque les compositions de l'invention sont des poudres du visage ou du corps, elles comprennent généralement au moins 70% en poids, de préférence de 70 à 99,9% en poids, par rapport au poids total de la composition, de phase pulvérulente.

La phase liante peut alors représenter jusqu'à 30% en poids, de préférence de 0,1 à 23% en poids, et préférentiellement encore de 3 à 20% en poids, par rapport au poids total de la composition.

La composition selon l'invention peut se présenter sous la forme d'une poudre, par exemple compacte, libre, pressée ou encore coulée. Dans le cas d'une poudre libre par exemple, la phase liante peut représenter jusqu'à 15% en poids, par rapport au poids total de la composition, de préférence de 1 à 5% en poids, par rapport au poids total de la composition. Pour une poudre compacte, la teneur en phase liante peut représenter de 1 à 30% en poids, de préférence de 5 à 20% en poids, par rapport au poids total de la composition.

Lorsque les compositions selon l'invention sont des fonds de teint, des rouges-à-lèvres, des mascaras, des eye-liners ou encore des produits de maquillage du corps, la phase liante peut représenter de 50% à 99,9% en poids, par rapport au poids de la composition finale. La composition peut alors se présenter sous la forme d'un produit fluide, d'un produit coulé, d'une crème, d'un stick, etc...

Les compositions selon l'invention peuvent également comprendre entre 30% et 50% de phase liante. On obtient alors un produit qui peut à la fois jouer le rôle d'une poudre et d'un fond de teint. Un tel produit est particulièrement avantageux :
il est extrêmement pratique car il est compact, donc facile à transporter. De plus, il allie à la fois les qualités cosmétiques d'une poudre, telles que la tenue et l'effet poudré obtenu sur la peau et celles d'un fond de teint, telles que le confort, l'apport d'hydratation, le glissant et la facilité d'application. Un tel produit est crémeux, il apporte un effet fraîcheur très agréable, ne procure aucun effet de dessèchement ou de tiraillement. Il peut également être utilisé comme produit de maquillage du corps.

Les compositions selon l'invention peuvent être préparées selon les méthodes connues de préparation des poudres, des émulsions, des coulés, des sticks cosmétiques.

Il est également possible de réaliser des fonds de teint, des rouges-à-lèvres, des mascaras, des eye-liners, des produits de maquillage du corps à volonté à l'aide d'un kit comprenant plusieurs récipients séparés. Un des récipients comprend la phase liante préalablement préparée. D'autres récipients peuvent comprendre des pigments de couleurs différentes, des actifs sous forme pulvérulente, des charges ou des nacres ou des paillettes. Chaque récipient peut ne comprendre qu'un seul type de composé ou au contraire un mélange préalablement réalisé. Il suffit pour réaliser le produit de maquillage de mettre quelques grammes de phase liante dans le creux de la main puis d'y ajouter les composés pulvérulents en les mélangeant à la phase liante à l'aide du doigt. Après quelques mouvements circulaires (une vingtaine par exemple), on obtient une composition homogène, dans laquelle les composés pulvérulents sont particulièrement bien dispersés, très facile à appliquer sur les matières kératiniques telles que la peau ou les muqueuses. Le maquillage obtenu est homogène et uniforme et extrêmement confortable.

Ainsi, la présente invention porte également sur un procédé de maquillage des matières kératiniques des êtres humains caractérisé en ce qu'il comprend les étapes suivantes :
- a°) le mélange d'au moins un composé pulvérulent et d'une quantité efficace de phase liante comprenant une dispersion de particules de gel cubique jusqu'à obtention d'une composition homogène,
- b°) l'application sur lesdites matières kératiniques de la composition obtenue en a°).

Le mélange peut se faire par exemple au creux de la main ou dans tout récipient de toute contenance. Par quantité efficace, on entend la quantité nécessaire de phase liante pour disperser les composés pulvérulents et obtenir une composition homogène. L'homme du métier est apte à déterminer cette quantité en fonction de la quantité de composés pulvérulents utilisée. Par composition homogène, on entend une composition dans laquelle les composés pulvérulents sont finement et régulièrement dispersés, la composition finale ne formant ni aggrégats ni grumeaux. De préférence, si A est la masse en gramme de phase liante utilisée et si B est la masse en gramme de composés pulvérulents utilisés, on privilégiera un rapport A/B allant de 100 :1 à 1 :1.

Le mélange de la dispersion de particules de gel cubique et des composés pulvérulents peut être réalisé à l'aide de tout appareil mélangeur, comme par exemple un « Moritz » (turbine microdisperseuse, vitesse maximale pendant 10 minutes) ou un « Ultra Turax » (avec ou sans pales raclantes) ou tout simplement avec un doigt.

Dans une forme préférée de réalisation de l'invention, le mélange de l'étape a°) se fait avec un doigt au creux de la main. On peut par exemple déposer environ 0,2 g à 1 g (gramme) de phase liante dans la main et ajouter environ 0,01 g à 1 g de composés pulvérulents. On mélange alors le tout à l'aide d'un doigt jusqu'à obtention d'une composition homogène. Selon que l'on désirera obtenir plutôt un fond de teint clair, foncé, de soin, on mélangera avec la phase liante des oxydes de titane ou des oxydes de fer ou encore des actifs de soin sous forme pulvérulente. Pour un rouge-à-lèvre, on peut par exemple utiliser des pigments rouges comme certains oxydes de fer.

Bien sûr, on peut également réaliser les fonds de teint ou les autres produits de maquillage selon l'invention par les méthodes classiques de préparation de ces produits, à savoir par exemple par prédispersion des pigments dans un corps gras approprié, broyage et agitation par exemple à l'aide d'un homogénéiseur de type « Virtis ® ».

L'invention est illustrée plus en détails dans les exemples suivants.

Dans les exemples suivants, les quantités sont données en pourcentage en poids par rapport au poids total de la composition.

### EXEMPLE 1 :

La Demanderesse a réalisé les compositions sous forme de poudres suivantes (les quantités sont données en pourcentage de poids par rapport au poids total de la composition) :

### Phase A :

- talc X %
- oxydes de fer 2,74 %
- poudre de Nylon 10 %
- oxyde de titane 1%
- conservateur 0,2 %

### Phase B :

- phase liante (dispersion de particules de gel cubique) Y %
avec :

| | X % | Y % |
|---|---|---|
| Composition 1 | 77,06 | 9 |
| Composition 2 | 74,06 | 12 |
| Composition 3 | 71,06 | 15 |

La composition de la phase liante (dispersion de particules de gel cubique) est la suivante (les quantités sont données en pourcentage de poids par rapport au poids total de la phase liante) :
- myristate d'isopropyle 1,64%
- huile de ricin 2,46%
- huile de vaseline 12,36%
- lanoline liquide 1,26%
- eau 70,95%
- imidazolinyl urée 0,3%
- glycérine 5%
- sel monosodique de N-stéaroyle-L-acide glutamique commercialisé sous la dénomination « Acylglutamate HS-11 » par la société Ajinomoto 0,03%
- phytantriol 2,97%
- vaseline 2,28%
- chlorphénésine 0,25%
- monopalmitate de sorbitane oxyéthyléné (40 OE) 0,5%

Cette composition est réalisée de la manière suivante : une dispersion aqueuse de particules de gel cubique est obtenue par mélange de 2,97 g de phytantriol et 0,03 g de sel monosodique de N-stéaroyle-L-acide glutamique et de 1,28 g d'eau, auquel on ajoute 75,17 g d'une solution aqueuse comprenant 0,5 g de monopalmitate de sorbitane oxyéthyléné et 5 g de glycérine. Le mélange est alors prédispersé puis homogénéisé à température ambiante, à l'aide d'un homogénéiseur de type « Virtis® » à 35 000 tr/min pendant 5 minutes, cette agitation étant répétée 4 fois.

A la dispersion aqueuse de particules de gel cubique obtenue, on ajoute alors les conservateurs (chlorphénésine et imidazolinyl urée) et les corps gras (myristate d'isopropyle, huile de ricin, huile de vaseline, lanoline liquide et vaseline). Après agitation à température ambiante à l'aide d'un homogénéiseur de type « Virtis ® » à 35 000 tr/min pendant 5 minutes, cette agitation étant répétée 5 fois, on obtient une dispersion stable et homogène.

Pour chaque composition 1 à 3, on prépare d'abord un mélange des composés de la phase A (composés pulvérulents) dans un mélangeur à turbine de type « Baker-Perkins ». Puis on y ajoute la phase B (phase liante) sous agitation. Les deux phases sont mélangées jusqu'à obtention d'un produit homogène. La température du mélange est gardée à 35°C.

On obtient des poudres présentant une bonne cohésion des composés pulvérulents. Ces compositions procurent un effet fraîcheur à l'application sur la peau.

### EXEMPLE 2 :

La demanderesse a réalisé la dispersion de particules de gel cubique suivante :
- isoparaffine hydrogénée 7,8%
- néopentanoate d'isostéaryle 2,6%
- conservateurs 0,61%
- parfum 0,1%
- sel penta-sodique de l'acide éthylène diamine tetra méthylène phosphonique dans l'eau à 33% 0,1%
- hydroxy éthyl cellulose alkyle 1%
- cyclohexa diméthylsiloxane 11,6%
- phytantriol 2,97%
- eau déminéralisée 69,19%
- glycérine 3%
- sel monosodique de N-stéaroyle-L-acide glutamique commercialisé sous la dénomination « Acylglutamate HS-11 » par la société Ajinomoto 0,03%
- monopalmitate de sorbitane oxyéthyléné (40 OE) 1%

Cette composition est réalisée de la manière suivante : une dispersion aqueuse de particules de gel cubique est obtenue par mélange de 2,97 g de phytantriol et 0,03 g de sel monosodique de N-stéaroyle-L-acide glutamique et de 1,28 g d'eau, auquel on ajoute 57,91 g d'une solution aqueuse comprenant 1 g de monopalmitate de sorbitane oxyéthyléné et 3 g de glycérine. Le mélange est alors prédispersé puis homogénéisé à température ambiante, à l'aide d'un homogénéiseur de type « Virtis® » à 35 000 tr/min pendant 5 minutes, cette agitation étant répétée 4 fois.

A la dispersion aqueuse de particules de gel cubique obtenue, on ajoute alors le parfum et les corps gras (isoparaffine hydrogénée, néopentanoate d'isostéaryle, cyclohexa diméthylsiloxane). Le mélange est alors homogénéisé à température ambiante à l'aide d'un homogénéiseur haute pression de type « Soavi® » par 4 passages à 600 bars.

On y ajoute ensuite une solution obtenue par mélange de l'hydroxy éthyl cellulose alkyle et des conservateurs avec 14 g d'eau déminéralisée. On homogénéise le mélange à température ambiante à l'aide d'un agitateur à palette de type « Heidolph RZR 50 ® » à 50 tr/min pendant 30 minutes.

On obtient ainsi une dispersion de particules de gel cubique stable et homogène.

On prend 0,36 g de cette dispersion dans le creux de la main et on y ajoute 0,03 g de pigment pur (oxyde de fer jaune). On mélange avec l'index par un mouvement circulaire (20 tours environ). On obtient un fond de teint sous la forme d'une crème fluide dans laquelle les pigments sont très bien dispersés.

On applique ensuite directement cette crème sur le visage, on étale et on obtient un maquillage parfaitement homogène et uniforme.

### EXEMPLE 3 :

On stocke la dispersion de particules de gel cubique de l'exemple 2 dans un flacon. On stocke dans des récipients séparés des oxydes de fer jaune, des oxydes de fer noirs, des oxydes de fer rouges, des oxydes de titane blancs, des nacres, des paillettes. On stocke dans d'autres récipients séparés des actifs sous forme pulvérulente et des charges. On obtient ainsi un kit de maquillage permettant de réaliser chez soi son propre fond de teint par simple mélange au creux de la main de dispersion de particules de gel cubique et des composés pulvérulents (pigments et/ou charges et/ou nacres et/ou paillettes et/ou actifs) choisis.

### EXEMPLE 4 :

La Demanderesse a réalisé la composition suivante (les quantités sont données en pourcentage de poids par rapport au poids total de la composition) :
- talc 46,06 %
- oxydes de fer 2,74 %
- poudre de Nylon 10 %
- oxyde de titane 1%
- conservateur 0,2 %
- dispersion de particules de gel cubique 40 %

La dispersion de particules de gel cubique utilisée est la même que dans l'exemple 1.

On obtient un produit dont la texture est plus compacte qu'une crème mais qui n'est pas une poudre. Ce produit peut être utilisé à la fois comme un fond de teint et comme une poudre pour le visage. Il permet d'obtenir un effet poudré sur la peau tout en apportant un effet fraîcheur à l'application. Il est confortable et facile à appliquer.

## Revendications

1. Composition de maquillage comprenant une phase pulvérulente et une phase liante, **caractérisée par le fait que** la phase liante comprend une dispersion de particules de gel cubique.

2. Composition selon la revendication 1 telle que la phase pulvérulente comprend des pigments et/ou des nacres et/ou des charges et/ou des paillettes et/ou leurs mélanges.

3. Composition selon la revendication 2 telle que les pigments sont choisis parmi les dioxydes de titane, de zirconium ou de cérium, les oxydes de zinc, de fer ou de chrome, les nanotitanes, les nanozincs, le bleu ferrique, le noir de carbone, les laques comme les sels de calcium, de baryum, d'aluminium ou de zirconium, de colorants acides tels que les colorants halogéno-acides, azoïques ou anthraquinoniques, les pigments enrobés par des composés siliconés tels que des polydiméthylsiloxanes et/ou par des polymères, notamment des polyéthylènes.

4. Composition selon la revendication 2 ou 3 telle que les pigments sont présents à une teneur allant de 0,05 à 80 %, de préférence allant de 0,5 à 50 %.en poids, par rapport au poids total de la composition.

5. Composition selon l'une quelconque des revendications 2 à 4 telle que les charges sont choisies parmi le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges et les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone.

6. Composition selon l'une quelconque des revendications 2 à 5 telle que les charges sont présentes à une teneur allant de 0,05 à 99 % en poids, par rapport au poids total de la composition.

7. Composition selon l'une quelconque des revendications 2 à 6, **caractérisée par le fait que** la phase pulvérulente comprend des nacres.

8. Composition selon la revendication précédente, **caractérisée par le fait que** les nacres sont présentes à une teneur allant de 0,05 à 80% en poids, de préférence allant de 2 à 50% en poids, par rapport au poids total de la composition.

9. Composition selon l'une quelconque des revendications 2 à 8 telle que la phase pulvérulente comprend des paillettes.

10. Composition selon l'une quelconque des revendications précédentes telle que la phase pulvérulente est présente à une teneur d'au moins 0,1% en poids, de préférence encore d'au moins 2% en poids, par rapport au poids total de la composition.

11. Composition selon l'une quelconque des revendications précédentes telle que la dispersion de particules de gel cubique est sous la forme d'une dispersion, à phase continue aqueuse, de particules de gel cubique à base de phytantriol comprenant :
(a) de 0,1 à 15 % en poids de 3,7,11,15-tétraméthyl 1,2,3-hexadecanetriol ou phytantriol par rapport au poids total de phase liante, et
(b) de 0,1 à 3 % en poids d'un agent dispersant et stabilisant par rapport au poids total de la phase liante, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

12. Composition selon l'une quelconque des revendications 1 à 10 telle que la dispersion de particules de gel cubique est une composition sous forme d'une dispersion comprenant :
(α) de 60 à 98 % en poids d'une phase aqueuse, et
(β) de 2 à 40 % en poids d'une phase huileuse,
ladite phase huileuse étant dispersée dans ladite phase aqueuse et stabilisée à l'aide de particules de gel cubique, lesdites particules étant essentiellement formées par :
(i) 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et
(ii) 0,05 à 3 % en poids par rapport au poids total de la composition d'un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

13. Composition selon la revendication 12 telle que la proportion pondérale relative en composé (i) par rapport au poids de la phase huileuse est comprise entre 0,02/1 et 1/1, et de préférence comprise entre 0,05/1 et 0,5/1.

14. Composition selon la revendication 12 telle que la proportion pondérale relative en composé (i) par rapport au poids dudit agent dispersant et stabilisant est comprise entre 2 et 200, et de préférence inférieure ou égale à 50.

15. Composition selon l'une quelconque des revendications 11 à 14 telle que l'agent dispersant et stabilisant est choisi parmi les alkyl ou alcényl éthers ou esters de polyol, les amino-acides N-acylés et leurs dérivés et les peptides N-acylés par un radical alkyle ou alcényle, et leurs sels, les alkyl ou alcényl éthers ou esters sulfates, leurs dérivés et leurs sels, les alkyl ou alcényl éthers ou esters gras polyoxyéthylénés, les acides alkyl ou alcényl carboxyliques polyoxyéthylénés et leurs sels, les N-alkyl ou alcényl bétaïnes, les alkyl ou alcényl triméthylammonium et leurs sels, et leurs mélanges.

16. Composition selon l'une quelconque des revendications précédentes telle que les particules de gel cubique comprennent en outre de 0,0005 % à 5 % en poids et de préférence de 0,001 % à 2 % en poids d'un lipide amphiphile ionique non hydrosoluble.

17. Composition selon l'une quelconque des revendications précédentes, **caracté risée ence que** les particules peuvent contenir un principe actif hydrophile ou un principe actif lipophile.

18. Composition selon l'une quelconque des revendications 12 à 17 telle que la phase huileuse comprend au moins une huile d'origine végétale, animale, minérale ou de synthèse.

19. Composition selon la revendication 18 telle que les huiles sont choisies parmi l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique. l'isohexadécane, le polydécène et le polyisobutène, des alcools gras tels que l'octyldodécanol, le cétanol, l'alcool stéarylique ou l'alcool oléique, des esters tels que les glycérides d'acides gras essentiels, les triglycérides des acides caprique et acprylique et leurs mélanges, et des esters d'alcools gras et d'acide gras linéaire ou ramifié comme l'huile de purcellin, des huiles de silicone de type linéaire telles que le polydiméthylsiloxane, de type cyclique telles que le cyclopentadiméthylsiloxane et de type organomodifié telles que le polyphényltriméthylsiloxane et le polydiméthylsiloxane oxyéthyléné oxypropyléné et leurs mélanges.

20. Composition selon l'une quelconque des revendications précédentes telle qu'elle comprend au moins un agent gélifiant.

21. Composition selon la revendication 20, **caractérisée en ce** l'agent gélifiant est présent à une teneur allant de 0,1 à 2% en poids par rapport au poids total de la phase liante.

22. Composition selon l'une quelconque des revendications précédentes **caractérisée par le fait que** la phase liante comprend en outre une huile volatile.

23. Composition selon l'une quelconque des revendications précédentes, **caractérisée par le fait qu'**elle se présente sous la forme de fard à paupières, de poudre du visage et du corps, d'anticerne, de fond de teint, de produit de maquillage du corps, de rouge-à-lèvres, de mascara, d'eye-liner, de produit solaire ou autobronzant.

24. Composition selon l'une quelconque des revendications précédentes qui est une poudre du visage ou du corps et dans laquelle la phase liante représente jusqu'à 30% en poids, de préférence de 0,1 à 23% en poids, par rapport au poids total de la composition.

25. Composition selon l'une quelconque des revendications 1 à 23 qui est un fond de teint, un rouge-à-lèvre, un mascara, un eye-liner ou un produit de maquillage du corps et dans laquelle la phase liante représente de 50% à 99,9% en poids, par rapport au poids de la composition.

26. Composition selon l'une quelconque des revendications 1 à 22 telle qu'elle comprend entre 30 et 50% de phase liante.

27. Procédé cosmétique de maquillage ou de soin des matières kératiniques des êtres humains, en particulier de la peau et du corps, comprenant l'application sur ces matières d'une composition telle que définie à l'une quelconque des revendications 1 à 26.

28. Utilisation d'au moins une dispersion de particules de gel cubique, dans une composition de maquillage comprenant des composés pulvérulents, dans le but d'améliorer la dispersion desdits composés pulvérulents dans ladite composition.

29. Utilisation d'au moins une dispersion de particules de gel cubique, dans une composition de maquillage et/ou de soin, dans le but d'améliorer l'hydratation sur les matières kératiniques conférée par ladite composition.

30. Kit de maquillage comprenant d'une part un récipient comprenant une composition comprenant au moins une dispersion de particules de gel cubique et d'autre part au moins un récipient séparé comprenant un composé pulvérulent.

31. Procédé de maquillage des matières kératiniques des êtres humains **caractérisé en ce qu'**il comprend les étapes suivantes :
- a°) le mélange d'au moins un composé pulvérulent et d'une quantité efficace de phase liante comprenant une dispersion de particules de gel cubique de façon à obtenir une composition homogène,
- b°) l'application sur lesdites matières kératiniques de la composition obtenue en a°).

32. Procédé selon la revendication précédente, **caractérisé en ce que** le mélange de l'étape a°) se fait avec un doigt au creux de la main.

33. Procédé selon la revendication 31 ou 32, **caractérisé en ce que** le rapport A/B va de 100 :1 à 1 :1, dans lequel A est la masse en gramme de phase liante utilisée et B est la masse en gramme de composés pulvérulents utilisés.
